# EUROPÄISCHE PATENTANMELDUNG

(11) **EP 2 404 621 A1**
(43) Veröffentlichungstag der Anmeldung: **11.01.2012**
(21) Anmeldenummer: 11401539.9
(22) Anmeldetag: 06.07.2011
(51) Int. Cl.: A61L 9/22, H01T 23/00, C02F 1/74

(54) **Ionisationsvorrichtung**

(30) Priorität: 08.07.2010 DE 102010031111
(71) Anmelder: Luwatec GmbH Luft-und Wassertechnik, 06667 Weissenfels (DE)
(72) Erfinder: Fischer, Friedrich, 04107 Leipzig (DE)
(74) Vertreter: Wablat Lange Karthaus

(57) **Zusammenfassung**

Die Erfindung bezieht sich auf eine Ionisationsvorrichtung zur Entkeimung eines verunreinigten Mediums mit an Sauerstoffionen angereicherter Luft, bestehend aus einem isolierenden Gehäuse (1, 10) mit einer Luftzufuhr (30) und einer Luftabfuhr (31) und einer Spannungsquelle (20). Um die Ionisierungsvorrichtung einfach im Aufbau und flexibel in der Anwendung auszubilden, sieht die Erfindung vor, dass im Gehäuse (1, 10) ein Ventilator (5, 33) mit Flügeln (8) angeordnet ist, deren Enden (9) zur Ausbildung als Elektroden (3, 12) mit einem elektrisch leitfähigen Metall beschichtet sind, und dass die Elektroden (3, 12) an den Enden (9) der Flügel (8) mit im Gehäuse (1, 10) angeordneten, den Ventilator (5, 33) umgebenden Gegenelektrode (2, 11) zusammenwirken. (Fig. 1)

## Beschreibung

Die Erfindung bezieht sich auf eine Ionisierungsvorrichtung und auf ein Verfahren zur Entkeimung eines verunreinigten Mediums, wie Luft oder Wasser, durch mit Sauerstoffionen angereicherte Luft.

Eine Vorrichtung zur Verbesserung der Luftqualität mittels Ionisation ist aus DE 26 58 287 C3 bekannt. Bei dieser Ionisationsvorrichtung mit einer der Atmosphäre ausgesetzten Ionisierungselektrode, einer Gleichstromhochspannungsquelle, einem isolierenden Gehäuseteil, welches die Ionisierungselektrode umgibt, und einem am Gehäuse anliegenden, mit Masse verbundenen Leiter wird ein elektrischer Ladungsfluss vom isolierenden Gehäuseteil zur Masse ermöglicht. Dadurch werden negative Auswirkungen wie z.B. unangenehme Gerüche, stark verschmutze oder an negativen Ionen verarmte Luft abgeschwächt.

Aus DE 11 2004 002 362 B4 ist eine Vorrichtung zur Beeinflussung und Behandlung der Raumluft bekannt, bei der mittels eines Ionisationsapparates negative und positive Sauerstoffionen erzeugt werden. Die Ionisation erfolgt durch elektrische Entladung an Ionisationsröhren oder an Koronaentladungsröhren, wobei der Ionisationsapparat mit zeitlichen Folgen einer periodischen Wechselspannung betrieben wird.

Bei diesen Vorrichtungen ist der hohe technische Aufwand bei der Implementierung der Ionisationsvorrichtung mit zugehöriger Elektronik im jeweiligen Anwendungsfall nachteilig.

Der Erfindung liegt von daher die Aufgabe zu Grunde, eine Ionisationsvorrichtung und ein Verfahren zur Entkeimung eines Mediums mittels der Ionisationsvorrichtung zu schaffen, wobei die Ionisationsvorrichtung einfach im Aufbau und flexibel in der Anwendung sein soll.

Diese Aufgabe wird erfindungsgemäß mit einer Vorrichtung nach Anspruch 1 und einem Verfahren nach Anspruch 10 gelöst. Vorteilhafte Ausgestaltungen sind in den Unteransprüchen dargelegt.

Die Ionisationsvorrichtung zur Entkeimung eines verunreinigten Mediums mit an Sauerstoffionen angereicherter Luft umfasst ein die Ionisationsvorrichtung umgebendes isolierendes Gehäuse, eine Luftzufuhr, eine Luftabfuhr und eine Spannungsquelle, wobei im Gehäuse ein Ventilator mit Flügeln angeordnet ist, deren Enden zur Ausbildung als Elektroden mit einem elektrisch leitfähigen Metall beschichtet sind, und wobei die drehenden Elektroden an den Enden der Flügel mit im Gehäuse fest angeordneten, den Ventilator umgebenden Gegenelektroden zusammenwirken, wobei der von der im Gehäuse fest angeordneten Gegenelektrode umgebende Ventilator den ionisierten Luftstrom weiterleitet. Das den Ventilator umschließende Gehäuse ist hierbei als Röhre bzw. Kasten ausgebildet.

Die Enden der Flügel des Ventilators dienen hierbei als Elektrode, und die im Inneren des Gehäuses befestigte Gegenelektrode umschließt den Ventilator. Die Enden der mit einem elektrisch leitfähigen Metall beschichteten Flügel des Ventilators können Kontaktstifte aufweisen oder sind glatt oder sägezahnförmig ausgeführt.

Die Spannung wird hierbei über die den Ventilator umschließende Gegenelektrode oder über einen in der Rotationsachse des Ventilators angeordneten Schleifkontakt an die Elektrode des Ventilators angelegt. In der ersten Ausführungsform sind die als Elektrode dienenden Enden der Flügel des Ventilators mit Kontaktstiften versehen. Die Gegenelektroden zur Anlegung der Spannung sind im Inneren des Gehäuses befestigt.

Durch das erfindungsgemäße Verfahren zur Entkeimung eines verunreinigten Mediums mit positiven und negativen Sauerstoffionen, bei dem Sauerstoffionen erzeugt werden, die in das verunreinigte Medium eingeleitet werden, wird der Ventilator mit oder ohne eigenen Antrieb in Drehung versetzt, die Luft, welche über die Luftzufuhr eingeleitet wurde, ionisiert, aus der Vorrichtung über die Luftabfuhr abgeleitet und weitergeleitet, d.h. in das verunreinigte Medium eingeleitet.

Bei der Ausführung ohne eigenen Antrieb muss die Ionisationsvorrichtung in Systeme integriert werden, die über eine Luftströmung verfügen, so dass der Ventilator über die Luftströmung in Drehung versetzt wird. Diese Ausgestaltung erscheint speziell als zusätzliche Reinigungskomponente in einem größeren Be- bzw. Entlüftungssystem sinnvoll, da in diesem Fall eine entsprechend hohe Strömungsgeschwindigkeit der Luft vorhanden ist. Kommt die Ionisationsvorrichtung, z.B. in einem kleineren, abgeschlossenen Raum zur Anwendung, ist ein über einen Elektromotor angetriebener Ventilator vorteilhaft, da so die Reinigung der Luft in einem geschlossenen Kreislauf erfolgt. Die Luft wird dabei angesaugt, ionisiert und gereinigt und zurück in den Raum geleitet.

Die Ionisationsvorrichtung erzeugt durch die elektrostatische Aufladung inaktiver Sauerstoffmoleküle positiv und negativ geladene Sauerstoffionen, die zur Luft- und Wasserentkeimung eingesetzt werden. Die Ionisationsvorrichtung ist so konzipiert, dass die positiv und negativ geladenen Sauerstoffionen in der Vorrichtung erzeugt werden und dann über die Luftabfuhr in das zu behandelnde Medium eingeleitet werden. Ist das zu behandelnde Medium Luft, kann diese zur

Entkeimung/ Behandlung auch direkt durch die Ionisationsvorrichtung geleitet werden. Hierbei wird der in der Luft enthaltene Sauerstoff ionisiert. Die erzeugten Sauerstoffionen reagieren mit den organischen Stoffen in der zu behandelnden Luft und reduzieren diese deutlich.

Die Kombination eines Ventilators zum Transport der zu ionisierenden Luft und einer Ionisationsvorrichtung ermöglicht eine kompakte und vielfältig verwendbare Einheit, die ohne komplexe und im Betrieb sensible Bauteile auskommt. Somit lassen sich einfache Belüftungssysteme durch die erfindungsgemäße Ionisationsvorrichtung schaffen, um gleichermaßen eine Belüftung und Reinigung der Luft bzw. des Wassers zu bewerkstelligen. Die Vorrichtung kann mit einem Ausströmsystem gekoppelt werden, das im zu behandelnden Wasser integriert wird und über das die mit Sauerstoffionen angereicherte Luft ins zu behandelnde Wasser induziert wird.

Die Erfindung wird nachfolgend anhand mehrerer Ausführungsformen der Ionisationsvorrichtung näher erläutert, die sich in der Art der Erzeugung des elektrostatischen Feldes, durch welches die zu ionisierende Luft, bzw. wenn das zu reinigende Medium Luft ist, gegebenenfalls auch direkt das Medium, geleitet wird, und der Ausführung der Enden der Flügel unterscheiden. Es werden jeweils Ausführungsformen mit und ohne eigenem Antrieb sowie Ausführungsformen mit und ohne Isolator beschrieben. Es zeigt:
Fig. 1 die Ionisationsvorrichtung in der ersten Ausführungsform mit Kontaktstiften an den Enden der Flügel und mit alternierender Elektrode und fester Gegenelektrode,
Fig. 2 a, b die in Fig. 1 dargestellte Ionisationsvorrichtung in Vorderansicht als Röhre bzw. als Kasten,
Fig. 3 die Ionisationsvorrichtung nach Fig. 1 mit über die Ventilatorachse angeschlossener Elektrode,
Fig. 4a, b die in Fig. 3 dargestellte Ionisationsvorrichtung in Vorderansicht als Röhre bzw. als Kasten,
Fig. 5 die Ionisationsvorrichtung nach Fig. 1 mit alternierender Elektrode, feststehender Gegenelektrode und Antrieb des Ventilators mittels Elektromotor,
Fig. 6 a, b die in Fig. 5 dargestellte Ionisationsvorrichtung in Vorderansicht als Röhre bzw. als Kasten,
Fig. 7 die Ionisationsvorrichtung nach. Fig. 1 mit über die Ventilatorachse angeschlossener Elektrode und mit Antrieb des Ventilators mittels eines Elektromotors,
Fig. 8 die Ionisationsvorrichtung nach Fig. 1 mit sowohl alternierender Elektrode als auch feststehender Gegenelektrode und zwischen Elektrode und Gegenelektrode angeordnetem Isolator,
Fig. 9 a, b die in Fig. 8 dargestellte Ionisationsvorrichtung in Vorderansicht als Röhre bzw. Kasten,
Fig. 10 die Ionisationsvorrichtung nach Fig. 1 mit über die Ventilatorachse an die Spannungsquelle angeschlossener Elektrode und mit zwischen Elektrode und Gegenelektrode angeordnetem Isolator,
Fig. 11 die Ionisationsvorrichtung nach Fig. 1 mit drehender Elektrode und fester Gegenelektrode, mit Antrieb des Ventilators mittels Elektromotor und mit zwischen Elektrode und Gegenelektrode angeordnetem Isolator,
Fig. 12 die Ionisationsvorrichtung nach Fig. 1 mit über die Ventilatorachse an die Spannungsquelle angeschlossener Elektrode und zwischen drehender Elektrode und fester Gegenelektrode angeordnetem Isolator sowie einem Elektromotor zum Antrieb des Ventilators,
Fig. 13 die Ionisationsvorrichtung in der zweiten Ausführungsform mit sägezahnförmigen Enden der Flügel, mit alternierender Elektrode und mit fester Gegenelektrode,
Fig. 14 a, b die in Fig. 13 gezeigte Ionisationsvorrichtung in Vorderansicht als Röhre bzw. als Kasten,
Fig. 15 die Ionisationsvorrichtung nach Fig. 13 mit über die Ventilatorachse angeschlossener Elektrode und zwischen Elektrode und Gegenelektrode angeordnetem Isolator,
Fig. 16 die Ionisationsvorrichtung nach Fig. 13 mit sowohl alternierender Elektrode als auch fester Gegenelektrode und Antrieb des Ventilators mittels Elektromotor,
Fig. 17 die Ionisationsvorrichtung nach Fig. 13 mit über die Ventilatorachse angeschlossener Elektrode und Antrieb des Ventilators mittels Elektromotor,
Fig. 18 die Ionisationsvorrichtung nach Fig. 13 mit sowohl alternierender Elektrode als auch Gegenelektrode und zwischen Elektrode und Ventilator angeordnetem Isolator,
Fig. 19 die Ionisationsvorrichtung nach Fig. 13 mit über die Ventilatorachse angeschlossener Elektrode und zwischen Elektrode und Ventilator angeordnetem Isolator,
Fig. 20 die Ionisationsvorrichtung nach Fig. 13 mit alternierender Elektrode und Gegenelektrode, mit Antrieb des Ventilators mittels Elektromotor und zwischen Elektroden und Ventilator angeordnetem Isolator,
Fig. 21 die Ionisationsvorrichtung nach Fig. 13 mit über die Ventilatorachse angeschlossener Elektrode, Antrieb des Ventilators mittels Elektromotor und zwischen Elektroden und Ventilator angeordnetem Isolator,
Fig. 22 die Ionisationsvorrichtung in der dritten Ausführungsform mit glatten Flügelenden und mit sowohl alternierender Elektrode als auch Gegenelektrode,
Fig. 23 die Ionisationsvorrichtung in der vierten Ausführungsform mit einem Ventilator in Walzenform,
Fig. 24 die in Fig. 23 dargestellte Ausführungsform der Ionisierungsvorrichtung in einer Seitenansicht mit über die Ventilatorachse angeschlossener Elektrode,
Fig. 25 die in Fig. 23 dargestellte Ausführungsform der Ionisationsvorrichtung in einer Seitenansicht mit alternierender Elektrode und Gegenelektrode und
Fig. 26 eine Kombination der bevorzugten ersten und zweiten Ausführungsformen der Ionisationsvorrichtung nach Fig. 1 bis Fig. 22.

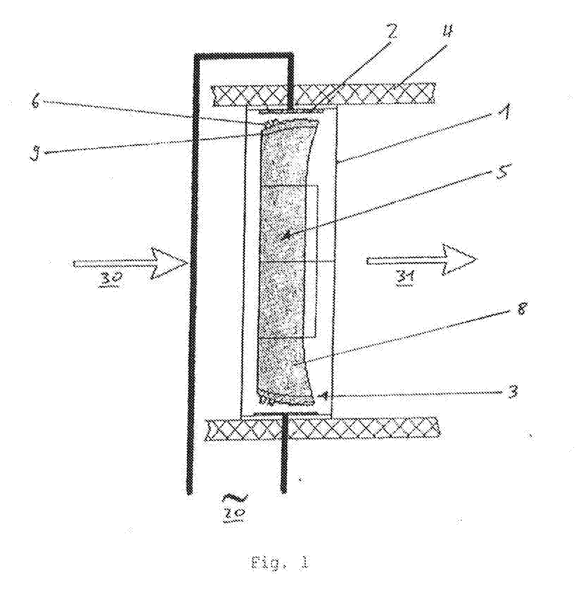

Die Fig. 1 zeigt die erste Ausführungsform der Ionisationsvorrichtung. Diese umfasst eine äußere Hülle 4, die ein Gehäuse 1 vollständig umschließt, das einen Ventilator 5 mit Elektrode 3 und eine diesen umschließende feststehende Gegenelektrode 2 beinhaltet. An den Enden 9 der Flügel 8 des Ventilators 5 befindet sich jeweils eine Elektrode 3, die aufgrund der Drehung des Ventilators 5 als alternierende Elektrode 3 ausgebildet ist. Die feststehende Gegenelektrode 2 ist an eine Spannungsquelle 20 angeschlossen. Die Enden 9 der Flügel 8 des Ventilators 5 sind mit Kontaktstiften 6 versehen, die sich in geringem Abstand zur gegenüber angeordneten Gegenelektrode 2 befinden.

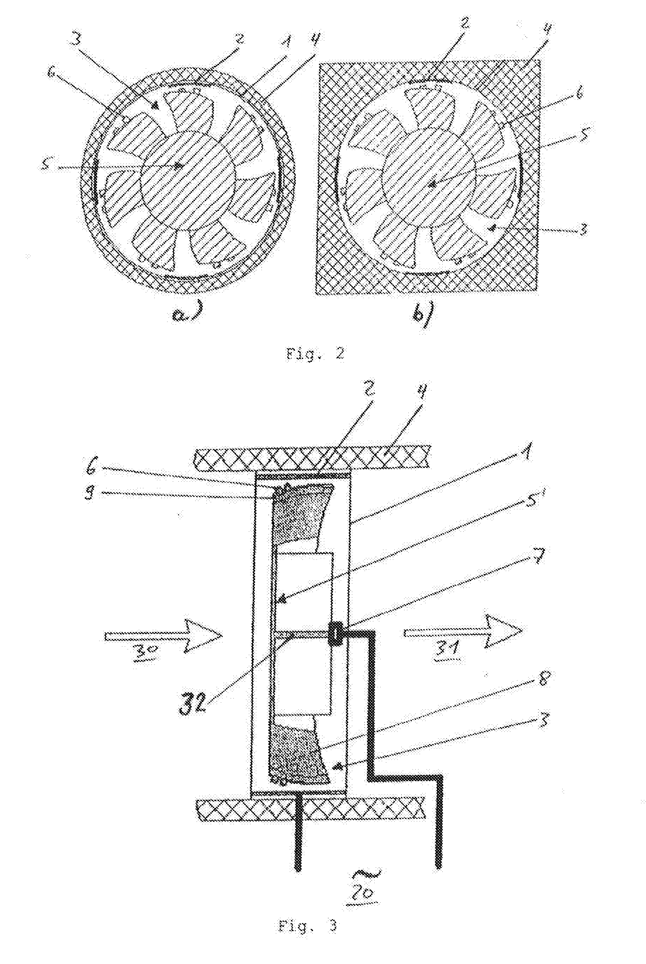

Die Fig. 2 a, b zeigt die Ionisationsvorrichtung nach Fig. 1 mit dem Ventilator 5 in zwei unterschiedlichen Ausführungen des Gehäuses 1 als Röhre bzw. als Kasten in Vorderansicht. Dieser ist kompakt ausgeführt und weist an den Enden 9 der Flügel 8 jeweils ein Paar Kontaktstifte 6 auf, die den elektrischen Kontakt zur Gegenelektrode 2 herstellen.

Die Drehung des Ventilators 5 bewirkt die Erzeugung eines elektrostatischen Feldes. Darin werden aus der angesaugten Luft positiv und negativ geladene Sauerstoffionen erzeugt, indem bei der hier vorliegenden Feldionisation Elektronen des Sauerstoffs durch ein ausreichend starkes Feld aus ihrer Bindung gelöst werden. Der Abstand zwischen der drehenden Elektrode 3 und der feststehenden Gegenelektrode 2 beeinflusst den Ionenabfluss, da sich der Abflusswiderstand für Ionen mit sich verringerndem Abstand zwischen der drehenden Elektrode 3 und der festen Gegenelektrode 2 verkleinert. Die so mit Sauerstoffionen angereicherte Luft wird in das zu behandelnde Medium geleitet.

Die Fig. 3 zeigt die modifizierte Ionisationsvorrichtung nach Fig. 1, wobei die ringförmige, den Ventilator 5 umschließende Gegenelektrode 2 starr am Gehäuse 1 befestigt und die Spannung zwischen der Elektrode 3 des Ventilator 5 und der Gegenelektrode 2 angelegt ist. Die Rotationsachse 32 des Ventilators 5 ist mit einem Schleifkontakt 7 versehen, über den die drehende Elektrode 3 mit der Spannungsquelle 20 verbunden ist. Der Ventilator 5 ist in einer leichteren Bauform mit sternförmigen Flügelaufnahmen ausgeführt und weist hier nicht dargestellte sägezahnförmig ausgebildete Enden 9 der Flügel 8 auf.

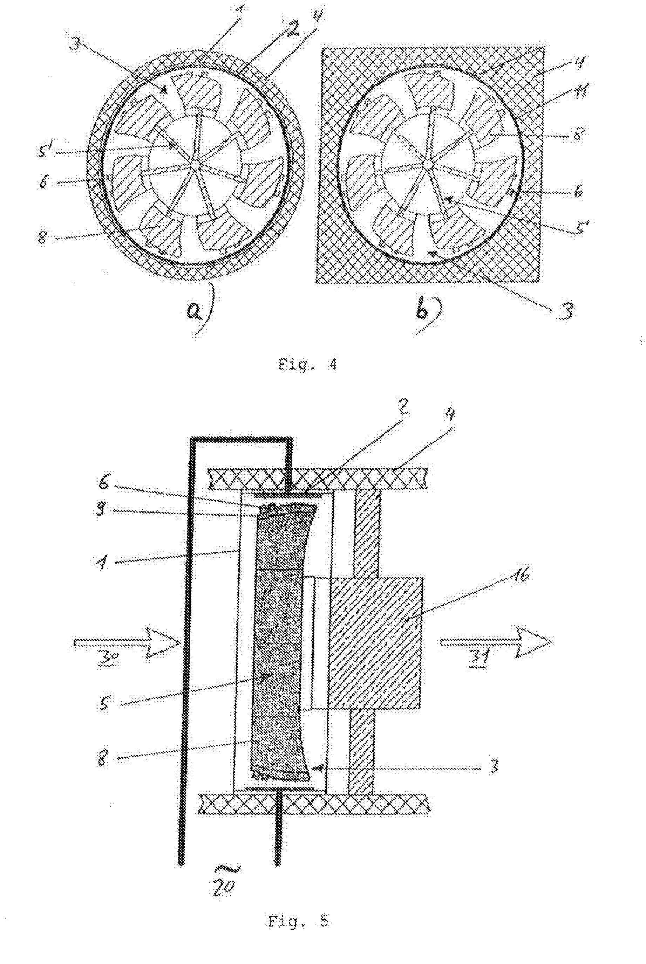

Die Fig. 4 a, b zeigt den in Fig. 3 dargestellten Ventilator 5 in Vorderansicht in den Ausführungsformen als Röhre bzw. als Kasten. Die Gegenelektrode 11 ist ringförmig mit geringem Abstand um die Enden 9 der Flügel 8 des Ventilators 5 herum angeordnet.

Der Ventilator 5 trägt die sich drehende Elektrode 3. Eine Anpassung der Frequenz des elektrischen Feldes ist durch den Einsatz eines Frequenzumrichters möglich, der aus der Spannung mit bestimmter Frequenz eine in Amplitude und Frequenz veränderbare Spannung generiert.

Die Fig. 5 zeigt die in Fig. 1 dargestellte modifizierte Ionisationsvorrichtung mit einem Elektromotor 16 zum Antrieb des Ventilators 5.

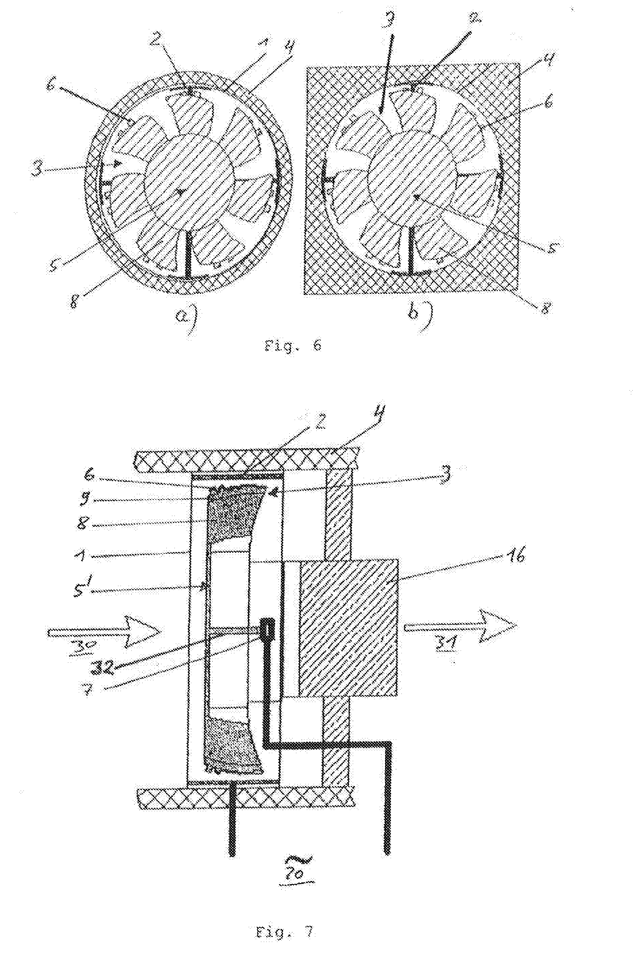

Die Fig. 6 a, b zeigt den in Fig. 5 dargestellten Ventilator 5 in Vorderansicht, der sich in einem als Röhre bzw. als Kasten ausgebildeten Gehäuse 1 befindet.

Die Fig. 7 zeigt die in Fig. 3 dargestellte modifizierte Ionisationsvorrichtung mit einem Elektromotor 16 zum Antrieb des Ventilators 5.

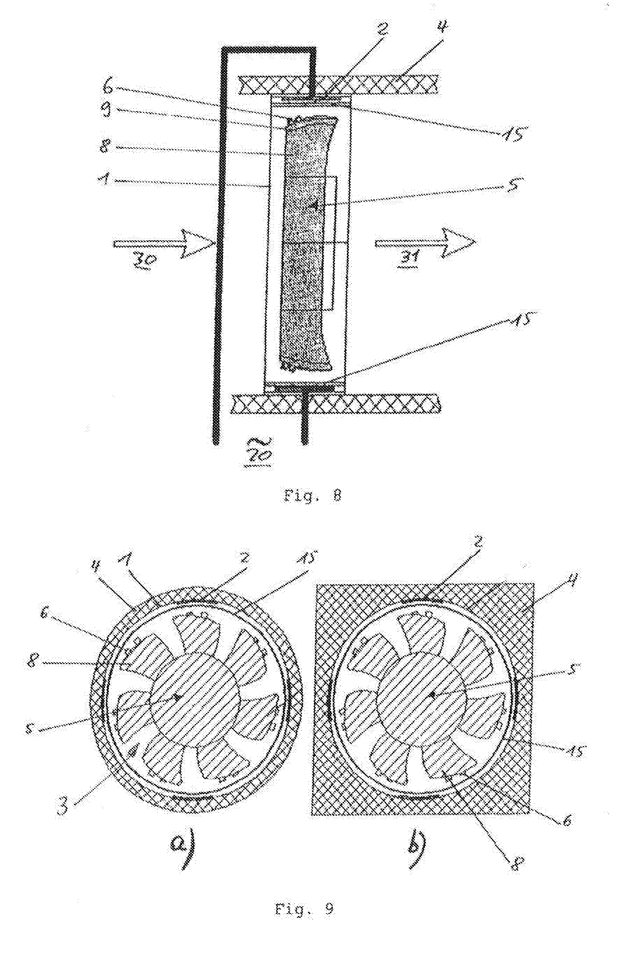

Die Fig. 8 zeigt die in Fig.1 dargestellte modifizierte Ionisationsvorrichtung mit einem zwischen Elektroden 3 und Ventilator 5 angeordneten Isolator 15. Auf der Oberfläche des Isolators 15 bilden sich Flächenladungen, die wiederum eine höhere Ionisationsrate zur Folge haben.

Die Fig. 9 a, b zeigt die in Fig. 8 dargestellte Ionisationsvorrichtung in Vorderansicht als Röhre bzw. als Kasten.

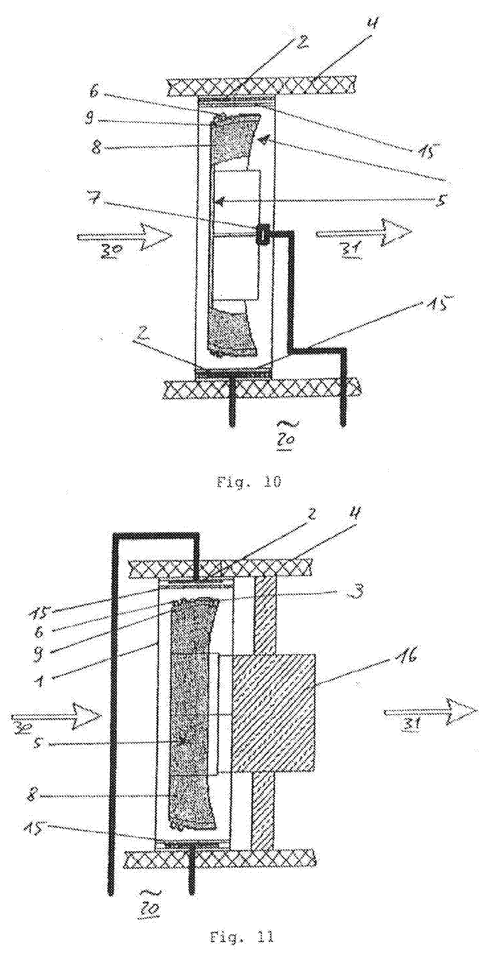

Die Fig. 10 zeigt die Ionisationsvorrichtung mit einem zwischen den Elektroden 3 des Ventilator 5 und der festen Gegenelektrode 2 angeordneten Isolator 15. Die Rotationsachse 32 des Ventilators 5 ist über einen Schleifkontakt 7 mit der Spannungsquelle 20 verbunden.

Die Fig. 11 zeigt die Ionisationsvorrichtung mit Isolator 15 und Elektromotor 16 zum Antrieb des Ventilators 5.

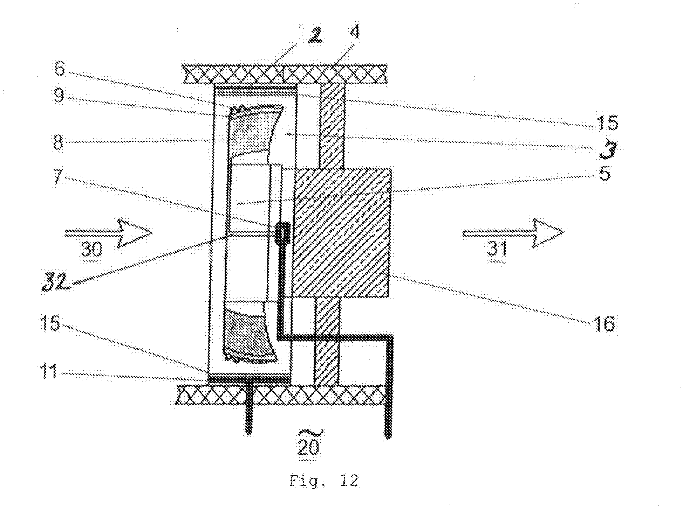

Die Fig. 12 zeigt die Ionisationsvorrichtung mit Isolator 15 und Elektromotor 16 zum Antrieb des Ventilators 5. Die Rotationsachse 32 des Ventilators 5 ist über den Schleifkontakt 7 mit der Spannungsquelle 20 verbunden.

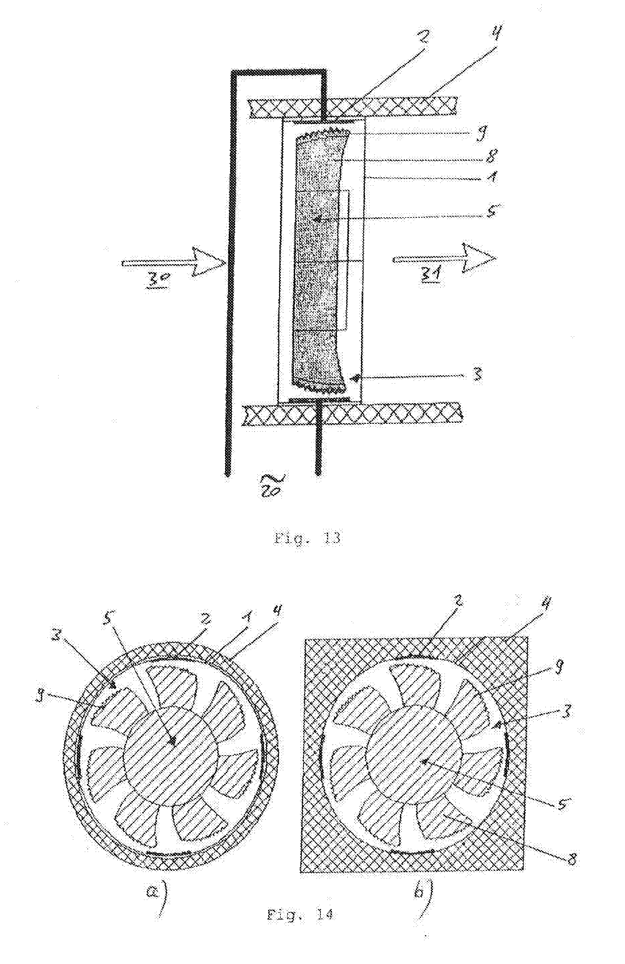

Die Fig. 13 zeigt die zweite Ausführungsform der Ionisationsvorrichtung mit sägezahnförmig ausgebildeten Enden 9 der Flügel 8 des Ventilators 5.

Die Fig. 14 a, b zeigt den in Fig. 13 dargestellten Ventilator 5 in Vorderansicht als Röhre bzw. Kasten. Die Gegenelektrode 2 ist ringförmig mit geringem Abstand um die Enden 9 der Flügel 8 des Ventilators 5 herum angeordnet. Diese weisen ein wellenförmiges Profil auf, das eine größere Ausbeute erzeugter Ionen aufgrund einer höheren Feldliniendichte zur Folge hat.

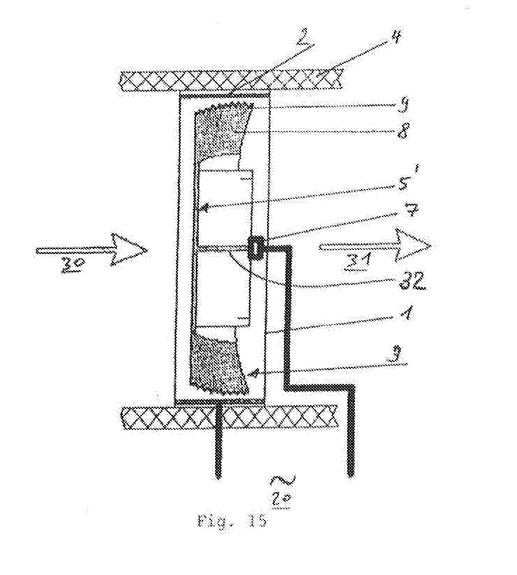

Die Fig. 15 zeigt die Ionisationsvorrichtung nach Fig. 13 mit mittels des Schleifkontaktes 7 über die Rotationsachse 32 des Ventilators 5 an die drehende Elektrode 3 angeschlossener Spannungsquelle 20.

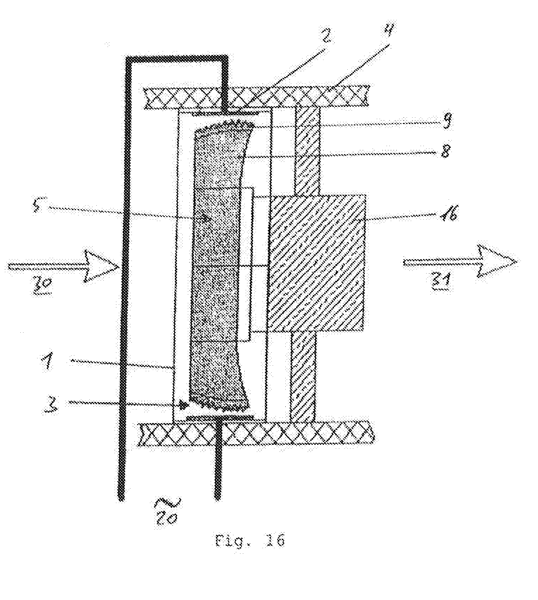

Die Fig. 16 zeigt die Ionisationsvorrichtung nach Fig. 13 mit Elektromotor 16 zum Antrieb des Ventilators 5.

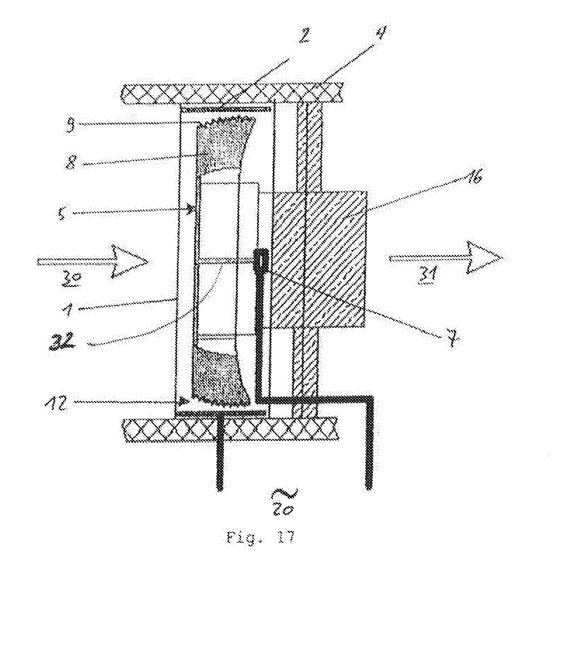

Die Fig. 17 zeigt die Ionisationsvorrichtung nach Fig. 13 mit Elektromotor 16 zum Antrieb des Ventilators 5 und mittels des Schleifkontaktes 7 über die Rotationsachse 32 des Ventilators 5 an die drehende Elektrode 3 angeschlossener Spannungsquelle 20.

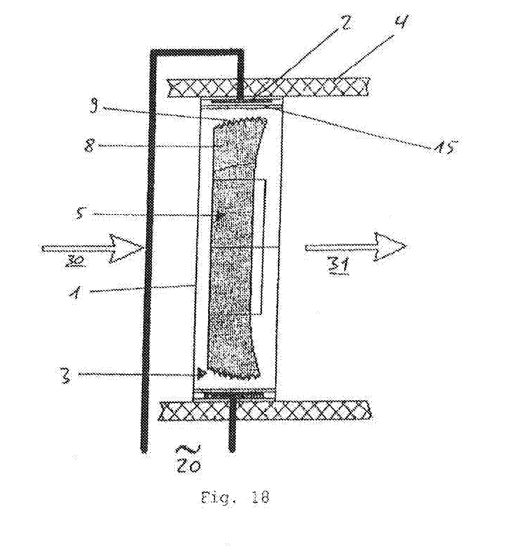

Die Fig. 18 zeigt die Ionisationsvorrichtung nach Fig. 13 mit zwischen den Gegenelektroden 2 und dem Ventilator 5 angeordnetem Isolator 15.

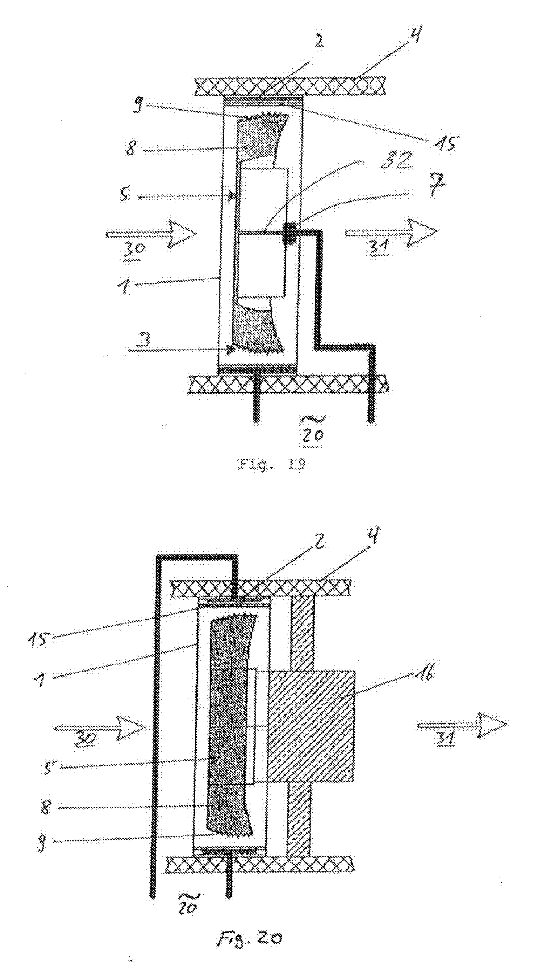

Die Fig. 19 zeigt die Ionisationsvorrichtung nach Fig. 13 mit zwischen den Gegenelektroden 2 und dem Ventilator 5 angeordnetem Isolator 15 und der mittels des Schleifkontaktes 7 über die Rotationsachse 32 des Ventilators 5 an die Spannungsquelle 20 angeschlossenen drehenden Elektrode 3.

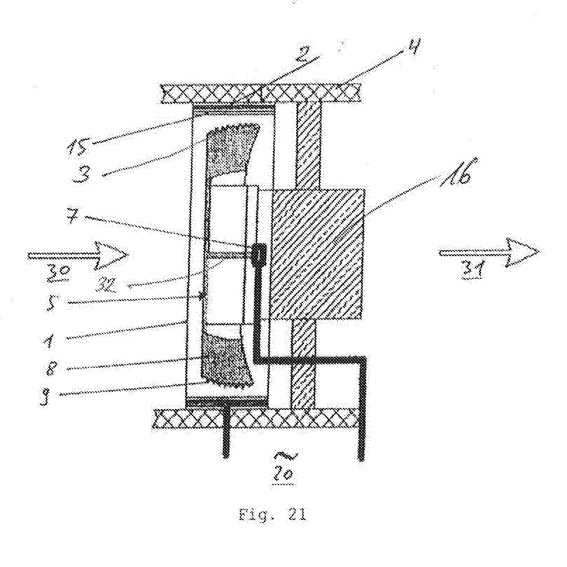

Die Fig. 20 und 21 zeigen die Ionisationsvorrichtung nach Fig.13 mit zwischen den Gegenelektroden 2 und dem Ventilator 5 angeordnetem Isolator 15 und mit dem Elektromotor 16 zum Antrieb des Ventilators 5 in den beiden Anschlussvarianten der Spannungsquelle 20 an die festen Gegenelektroden 2 bzw. die drehenden Elektroden 3.

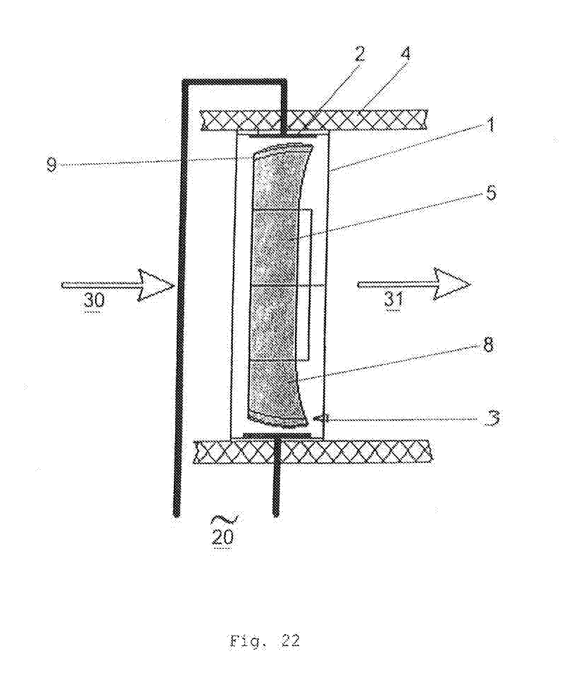

Die Fig. 22 zeigt die dritte Ausführungsform der Ionisationsvorrichtung mit glatten Enden 9 der Flügel 8. Alle weiteren Merkmale entsprechen der ersten Ausführungsform gemäß Fig. 1 bis Fig. 12. Es sind die oben genannten Anschlussvarianten, sowie Ausführungsformen mit und ohne Elektromotor und mit und ohne Isolator möglich.

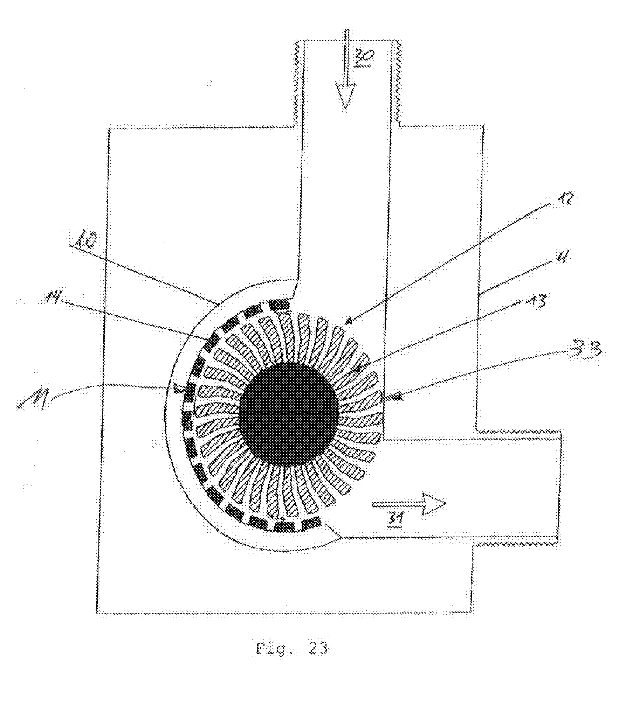

Die Fig. 23 zeigt die vierte Ausführungsform der Ionisationsvorrichtung im Querschnitt. Hier ist der Ventilator 33 als Walze mit engstehenden turbinenartigen Schaufeln 13 ausgebildet. Die feste Gegenelektrode 11 ist in längliche Segmente 14 unterteilt, deren Längsachsen parallel zur Rotationsachse 32 des Ventilators 33 verlaufen. Die in die länglichen Segmente 14 unterteilte Gegenelektrode 14 umschließt den Ventilator 33 zu über 50 Prozent. Die über die Luftzufuhr 30 angesaugte Luft tritt von oben in das Gehäuse 10 ein und strömt den Ventilator 33 radial an. Die ionisierte Luft verlässt das Gehäuse 10 horizontal durch die Luftabfuhr 31. Auch hier bewirkt die Drehung des Ventilators 33 die Erzeugung des elektrischen Feldes, dessen Frequenz durch die Variation der Drehzahl des Ventilators 33 bzw. durch die Anzahl der Elektroden 12 und Gegenelektroden 11 angepasst werden kann.

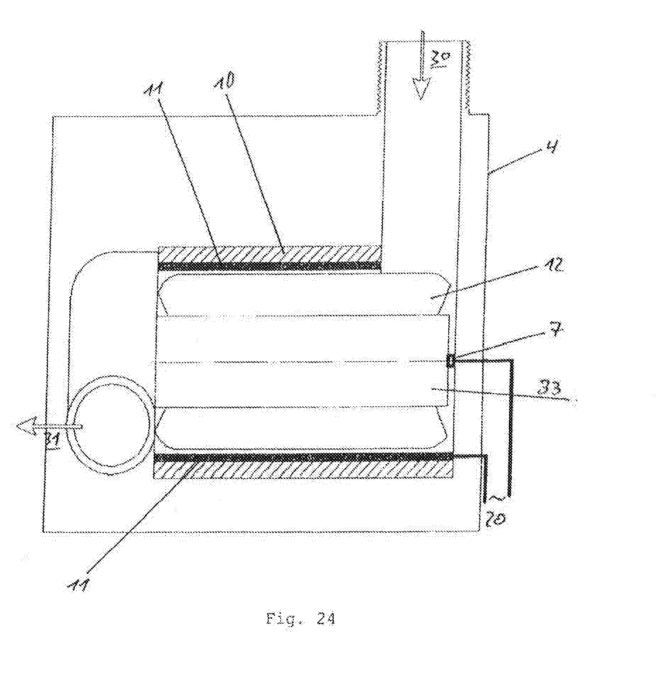

Die Fig. 24 zeigt die in Fig. 23 dargestellte vierte Ausführungsform der Ionisationsvorrichtung in einer Seitenansicht. Die feste Gegenelektrode 11 verläuft um den walzenförmigen Ventilator 33 über dessen gesamte Breite, abgesehen von einer kreisförmigen Aussparung im oberen Bereich der Luftzufuhr 30. Die ringförmige, den walzen-förmigen Ventilator 33 umschließende feste Gegenelektrode 11 ist starr am Gehäuse 10 befestigt und die Spannungsquelle 20 ist zwischen dem Ventilator 33 und festen Gegenelektrode 11 angelegt. Hierzu ist die Rotationsachse 32 des Ventilators 33 über den Schleifkontakt 7 mit der Spannungsquelle 20 verbunden.

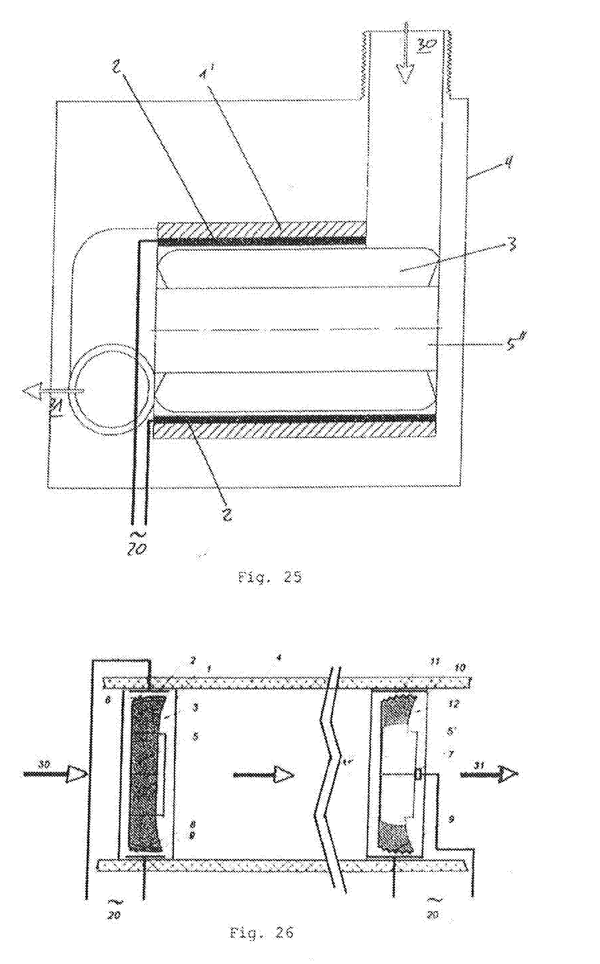

Fig. 25 zeigt die in Fig. 23 dargestellte vierte Ausführungsform der Ionisationsvorrichtung in einer Seitenansicht. An die Gegenelektrode 11 ist die Spannungsquelle 20 angeschlossen.

Die Fig. 26 zeigt eine Kombination bzw. Hintereinanderschaltung der ersten und zweiten Ausführungsformen der Ionisationsvorrichtung. Die beiden Ausführungsformen entsprechen den in Fig. 1 bis Fig. 22 dargestellten Ausführungsformen innerhalb einer beide Ausführungsformen umschließenden Hülle 4 in serieller Anordnung. Es sind auch andere Kombinationen möglich. Weitere, hier nicht gezeigte Kombinationen vorstehender Ausführungsformen sind möglich und müssen an den entsprechenden Einsatzzweck angepasst werden.

Die Flexibilität der erfindungsgemäßen Ionisationsvorrichtung zeigt sich dadurch, dass diese leicht an äußere Bedingungen wie zu entkeimendes Medium, Volumenstrom oder Keimbelastung, angepasst werden kann, indem die Anzahl der Ventilatorblätter und Elektroden oder die Größe der Ventilatorblätter bzw. der Abstand zwischen Gegenelektrode und Ventilatorblättern verändert wird. Dies erlaubt eine bauartbedingte Anpassung des gewünschten Ionenabflusses. Der Abflusswiderstand wird für Ionen umso geringer, je näher sich die festen Gegenelektroden 2, 11 an den Ventilatorblättern und damit an den drehenden Elektroden 3, 12 befinden. Zur Anpassung der Höhe der Spannung der Spannungsquelle 20 kann ein Isolator 15 eingesetzt werden. In Abhängigkeit der vorhandenen Keimbelastung und des geforderten Volumenstroms können mehrere, auch unter-schiedliche Ausführungsformen der Ionisationsvorrichtung in Reihe betrieben werden (siehe Fig. 26). Eine Änderung der Drehzahl ermöglicht ebenfalls eine direkte Variation des Volumenstroms.

Im Fall von Luft als zu reinigendem Medium wird die Ionisationsvorrichtung vorzugsweise direkt in ein Belüftungs- bzw. Reinigungssystem integriert. Soll verunreinigtes Wasser entkeimt werden, so ist eine an die Luftabfuhr anschließende Ankopplung an den wasserführenden Tank oder die entsprechende flüssigkeitsführende Leitung vorteilhaft. Die ionisierte Luft wird dann unter dem notwendigen Druck in die Flüssigkeit gepresst. Die Sauerstoffionen werden im Wasser verteilt und reagieren mit den dort vorhandenen organischen Stoffen und reduzieren diese.

### Bezugszeichen:

- 1: Gehäuse
- 2: Gegenelektrode (feststehend)
- 3: Elektrode (drehend bzw. alternierend)
- 4: Hülle
- 5: Ventilator
- 6: Kontaktstift
- 7: Schleifkontakt
- 8: Flügel (des Ventilators)
- 9: Ende (des Flügels)
- 10: Gehäuse
- 11: Gegenelektrode (feststehend)
- 12: Elektrode (drehend)
- 13: Schaufel (turbinenartig)
- 14: Segment (der Gegenelektrode)
- 15: Isolator
- 16: Elektromotor
- 20: Spannungsquelle
- 30: Luftzufuhr
- 31: Luftabfuhr
- 32: Rotationsachse
- 33: Ventilator

## Patentansprüche

1. Ionisationsvorrichtung zur Entkeimung eines verunreinigten Mediums mit an Sauerstoffionen angereicherter Luft, bestehend aus einem isolierenden Gehäuse (1, 10) mit einer Luftzufuhr (30) und einer Luftabfuhr (31) und einer Spannungsquelle (20), **dadurch gekennzeichnet, dass** im Gehäuse (1, 10) ein Ventilator (5, 33) mit Flügeln (8, 13) angeordnet ist, deren Enden (9) zur Ausbildung als Elektroden (3, 12) mit einem elektrisch leitfähigen Metall beschichtet sind, und dass die Elektroden (3, 12) an den Enden (9) der Flügel (8, 13) mit im Gehäuse (1, 10) angeordneten, den Ventilator (5, 33) umgebenden Gegenelektroden (2, 11) zusammenwirken.

2. Ionisationsvorrichtung nach Anspruch 1, **dadurch gekennzeichnet, dass** das Gehäuse (1, 10) als den Ventilator (5, 33) umschließende Röhre ausgebildet ist.

3. Ionisationsvorrichtung nach Anspruch 1, **dadurch gekennzeichnet, dass** das Gehäuse (1, 10) als den Ventilator (5, 33) umschließender Kasten ausgebildet ist.

4. Ionisationsvorrichtung nach einem der Ansprüche 1 bis 3, **dadurch gekennzeichnet, dass** die Enden (9) der Flügel (8, 13) des Ventilators (5, 33) Kontaktstifte (6) aufweisen.

5. Ionisationsvorrichtung nach einem der Ansprüche 1 bis 3, **dadurch gekennzeichnet, dass** die als Elektrode (3,12) ausgeführten Enden (9) der Flügel (8) des Ventilators (5) sägezahnförmig ausgebildet sind.

6. Ionisationsvorrichtung nach einem der Ansprüche 1 bis 3, **dadurch gekennzeichnet, dass** die als Elektrode (3,12) ausgeführten Enden (9) der Flügel (8) des Ventilators (5) eine glatte Oberfläche aufweisen.

7. Ionisationsvorrichtung nach einem der Ansprüche 1 bis 6, **dadurch gekennzeichnet, dass** in der Rotationsachse (32) des Ventilators (5) ein Schleifkontakt (7) zum Anschluss der an den Enden (9) der Flügel (8) des Ventilators (5) befindlichen Elektroden (2) an die Spannungsquelle (20) angeordnet ist.

8. Ionisationsvorrichtung nach einem der Ansprüche 1 bis 7, **dadurch gekennzeichnet, dass** der Ventilator (33) walzenförmig ausgebildet, mit engstehenden, die Elektroden (12) bildenden, turbinenartigen, länglichen, die Flügel (8) bildenden Schaufeln (13) versehen und von der in längliche Segmente (14) unterteilten Gegenelektrode (11) teilweise umschlossen ist.

9. Ionisationsvorrichtung nach einem der Ansprüche 1 bis 8, **dadurch gekennzeichnet, dass** zwischen den Gegenelektroden (2) und dem Ventilator (5) ein Isolator (15) angeordnet ist.

10. Verfahren zur Entkeimung eines verunreinigten Mediums durch eine Vorrichtung nach den Ansprüchen 1 bis 9, **dadurch gekennzeichnet, dass** der Ventilator (5, 33) in Drehung versetzt, Luft über die Luftzufuhr (30) eingeleitet, mittels der Vorrichtung ionisiert und aus der Vorrichtung über die Luftabfuhr (31) abgeleitet und in das zu behandelnde Medium eingeleitet wird.

11. Verfahren nach Anspruch 10, **dadurch gekennzeichnet, dass** der Ventilator (5, 33) durch die anströmende Luft (30) in Drehung versetzt wird.

12. Verfahren nach Anspruch 10, **dadurch gekennzeichnet, dass** der Ventilator (5, 33) mittels eines Elektromotors (16) angetrieben wird.
